# EUROPÄISCHE PATENTSCHRIFT

(11) **EP 2 678 411 B1**
(45) Veröffentlichungstag und Bekanntmachung des Hinweises auf die Patenterteilung: **29.03.2017**
(21) Anmeldenummer: 12703546.7
(22) Anmeldetag: 14.02.2012
(51) Int. Cl.: C11D 1/66, C11D 3/37, C11D 17/00, A61L 9/05, A61L 2/22, A61L 2/23

(54) **WC-GEL**
WC GEL
GEL WC

(30) Priorität: 25.02.2011 DE 102011004771
(43) Veröffentlichungstag der Anmeldung: 01.01.2014
(73) Patentinhaber: Henkel AG & Co. KGaA, 40589 Düsseldorf (DE)
(72) Erfinder: SCHIEDEL, Marc-Steffen, 40597 Düsseldorf (DE); GIESEN, Brigitte, 40625 Düsseldorf (DE); PLANTIKOW, Petra, 40627 Düsseldorf (DE); BELLOMI, Luca, 40627 Düsseldorf (DE); SCHEFFLER, Karl-Heinz, 40589 Düsseldorf (DE)
(86) Internationale Anmeldenummer: PCT/EP2012/052446
(87) Internationale Veröffentlichungsnummer: WO 2012/113671

(56) Entgegenhaltungen:
- EP-A1- 1 318 191
- DE-A1-102004 007 473
- US-A1- 2004 211 316
- AL-SABAGH A M ET AL: "Water-based non-ionic polymeric surfactants as oil spill dispersants", JOURNAL OF CHEMICAL TECHNOLOGY AND BIOTECHNOLOGY, JOHN WILEY & SONS LTD, UNITED KINGDOM, Bd. 74, 22. Mai 1999 (1999-05-22), Seiten 1075-1081, XP002163236, ISSN: 0268-2575, DOI: 10.1002/(SICI)1097-4660(199911)74:11<1075: :AID-JCTB125>3.3.CO;2-V

## Beschreibung

Die Erfindung betrifft eine haftende gelförmige oder pastöse Zusammensetzung zur Reinigung und/oder Beduftung eines WCs. Diese Zusammensetzung wird innen auf die WC-Keramik aufgetragen und erst nach mehreren Spülvorgängen abgespült.

WC-Duftspüler werden bereits seit langem zur Reinigung, Desinfektion und Beduftung von Toiletten verwendet. In ihrer ursprünglichen Form werden sie als feste Blocks unter dem Beckenrand (sogenannte Rimblocks) sowie im Wasserkasten (In-tank-Blocks oder cistern blocks) eingesetzt. Dabei haben in den letzten Jahren die Ästhetik und die Leistung eine immer größere Bedeutung erlangt. Dies führte beispielsweise zur Entwicklung gelförmiger oder flüssiger Duftspüler, die teilweise in Mehrkammerbehältnissen angeboten werden und so die Kombination eines Reinigungsmittels, welches bei Betätigung der WC-Spülung abgegeben wird, mit einer permanenten Raumbeduftung erlauben.

Herkömmliche feste, flüssige oder auch gelförmige Duftspüler werden dabei mittels entsprechender Vorrichtungen, der sogenannten WC-Körbchen, in die Spültoilette eingebracht. Diese WC-Körbchen werden von manchen Verbrauchern jedoch aus hygienischen Gründen abgelehnt. Zum einen kann im Verlauf der Lebensdauer des Produkts eine Besiedelung mit Keimen erfolgen, was zur Ausbildung eines unschönen Biofilms führen kann. Zum anderen wird ein Nachfüllen oder Austauschen des Körbchens aufgrund der notwendigen Berührung als unhygienisch oder gar ekelerregend empfunden, selbst wenn kein sichtbarer Biofilm an der Vorrichtung haftet. Und schließlich empfinden einige Verbraucher es auch als nachteilig, dass das Körbchen bei der WC-Reinigung mittels WC-Bürste verschoben werden kann.

Es wurden daher selbsthaftende Mittel entwickelt, die direkt auf die WC-Keramik appliziert werden und nach und nach abgespült werden. EP 1086199 B1 beschreibt beispielsweise ein festes oder pastöses, haftendes Sanitärmittel, welches Wasser, anionische und/oder nichtionische und/oder amphotere Tenside, Duftstoffe, einen Haftvermittler sowie ggf. weitere übliche Bestandteile umfasst, wobei der Haftvermittler ausgewählt sein soll aus der Gruppe bestehend aus Polyalkoxyalkanen, Cellulosen, Stärke, Alginaten, Diurethanen, Gelatine, Pectinen, Oleylaminen, Alkyldimethylaminoxiden, Stearaten, Natriumdodecylbenzolsulfonat, Agar-Agar, Gummi arabicum, Johannisbrotkernmehl, Polyacrylat, Polyvinylalkohol und Polyvinylpyrrolidon. Ähnliche Pasten werden auch in EP1318191 B1 beschrieben, in der jedoch der Haftvermittler aus der Gruppe der Oligo- oder Polyethylenoxid und/oder Oligo-und/oder Polypropylenoxid und/oder Oligo- und/oder Polybutylenoxid umfassenden Blockcopolymere ausgewählt sein soll.

Es wurde nun gefunden, dass eine haftende gelförmige oder pastöse Zusammensetzung formuliert werden kann, die mindestens ein Tensid aus der Gruppe bestehend aus Alkylpolyglycosiden, Amphoacetaten, Amphodiacetaten, Betainen, Fettalkoholethercarbonsäuren, Fettsäuresarcosinaten, Cocoamidopropylaminoxid, Aminopropionaten und Biotensiden, Parfüm und Wasser sowie als einzigen Haftvermittler einen Ester der Polyisobutenbernsteinsäure enthält. Dieses Produkt weist eine transparente und klare Ästhetik auf und besitzt nach der Applikation eine gute Formstabilität über den gesamten Abspülzyklus. Auch wenn die Zusammensetzung durch fehlende Betätigung der WC-Spülung über einen Zeitraum von einigen Stunden nicht von Wasser umspült wird, verändert es nicht die Form oder wird unansehnlich.

Gegenstand der Erfindung ist daher eine haftende gelförmige oder pastöse Zusammensetzung zur Reinigung und/oder Beduftung eines WCs, welche innen auf die WC-Keramik aufgetragen und erst nach mehreren Spülvorgängen abgespült wird, enthaltend mindestens ein Tensid aus der Gruppe bestehend aus Alkylpolyglycosiden, Amphoacetaten, Amphodiacetaten, Betainen, Fettalkoholethercarbonsäuren, Fettsäuresarcosinaten, Cocoamidopropylaminoxid, Aminopropionaten und Biotensiden, Parfüm, Wasser sowie als Haftvermittler einen Ester der Polyisobutenbernsteinsäure, die frei von weiteren Haftvermittlern und Tensiden ist

Diese gelförmige oder pastöse Zusammensetzung wird vorteilhafterweise mit einem Applikator aufgetragen. In einer Ausführungsform werden zwei gelförmige oder pastöse Formulierungen in einen Zweikammerapplikator eingebracht, wie er auch in der Patentanmeldung DE 102010028352.5 beschrieben wird.

Stoffe, die auch als Inhaltsstoffe von kosmetischen Mitteln dienen, werden nachfolgend ggf. gemäß der *International Nomenclature Cosmetic Ingredient* (INCI)-Nomenklatur bezeichnet. Chemische Verbindungen tragen eine INCI-Bezeichnung in englischer Sprache, pflanzliche Inhaltsstoffe werden ausschließlich nach Linne in lateinischer Sprache aufgeführt, sogenannte Trivialnamen wie "Wasser", "Honig" oder "Meersalz" werden ebenfalls in lateinischer Sprache angegeben. Die INCI-Bezeichnungen sind dem International Cosmetic Ingredient Dictionary and Handbook - Seventh Edition (1997) zu entnehmen, das von The Cosmetic, Toiletry, and Fragrance Association (CTFA), 1101 17th Street, NW, Suite 300, Washington, DC 20036, USA, herausgegeben wird und mehr als 9.000 INCI-Bezeichnungen sowie Verweise auf mehr als 37.000 Handelsnamen und technische Bezeichnungen einschließlich der zugehörigen Distributoren aus über 31 Ländern enthält. Das *International Cosmetic Ingredient Dictionary and Handbook* ordnet den Inhaltsstoffen eine oder mehrere chemische Klassen (*Chemical Classes*)*,* beispielsweise *Polymeric Ethers,* und eine oder mehrere Funktionen (*Functions*), beispielsweise *Surfactants - Cleansing Agents,* zu, die es wiederum näher erläutert und auf die nachfolgend ggf. ebenfalls Bezug genommen wird.

Die Angabe *CAS* bedeutet, dass es sich bei der nachfolgenden Zahlenfolge um eine Bezeichnung des *Chemical Abstracts Service* handelt.
Im Rahmen der vorliegenden Erfindung stehen Fettsäuren bzw. Fettalkohole bzw. deren Derivate-soweit nicht anders angegeben - stellvertretend für verzweigte oder unverzweigte Carbonsäuren bzw. Alkohole bzw. deren Derivate mit vorzugsweise 6 bis 22 Kohlenstoffatomen, insbesondere 8 bis 20 Kohlenstoffatomen, besonders bevorzugt 10 bis 18 Kohlenstoffatomen, äußerst bevorzugt 12 bis 16 Kohlenstoffatomen, beispielsweise 12 bis 14 Kohlenstoffatomen. Erstere sind insbesondere wegen ihrer pflanzlicher Basis als auf nachwachsenden Rohstoffen basierend aus ökologischen Gründen bevorzugt, ohne jedoch die erfindungsgemäße Lehre auf sie zu beschränken. Insbesondere sind auch die beispielsweise nach der ROELENschen Oxo-Synthese erhältlichen Oxo-Alkohole bzw. deren Derivate mit vorzugsweise 7 bis 19 Kohlenstoffatomen, insbesondere 9 bis 19 Kohlenstoffatomen, besonders bevorzugt 9 bis 17 Kohlenstoffatomen, äußerst bevorzugt 11 bis 15 Kohlenstoffatomen, beispielsweise 9 bis 11, 12 bis 15 oder 13 bis 15 Kohlenstoffatomen, entsprechend einsetzbar

In der erfindungsgemäßen Zusammensetzung wird als Haftvermittler ein Ester der Polyisobutenbernsteinsäure eingesetzt, wie er in der Patentanmeldung EP 11150613.5 beschrieben wird. Unter Polyisobutenbernsteinsäure versteht man oligomere oder polymere Makromoleküle mit einem Oligomerrest bzw. Polymerrest, der von Isobuten abgeleitet ist und der an einem seiner Termini 1 oder 2 von Bernsteinsäure abgeleitete Reste, also Reste der Formel BS

-CH(COOH)CH₂COOH (BS)

und dementsprechend 2 oder 4 Carboxylgruppen aufweist, sowie Gemische davon.

Polyisobutenbernsteinsäuren können daher durch die folgende Formeln IIa und II b beschrieben werden:

PIB-CH(COOH)CH₂COOH (IIa)

PIB'-[CH(COOH)CH₂COOH]₂ (IIb)

wobei PIB in Formel IIa für einen einwertigen, von Polyisobuten abgeleiteten Oligomerrest bzw. Polymerrest und PIB' in Formel IIb für einen zweiwertigen, von Polyisobuten abgeleiteten Oligomerrest bzw. Polymerrest stehen.

In den erfindungsgemäß verwendeten Estern der Polyisobutenbernsteinsäure liegt wenigstens eine der Carboxylgruppen in Form des Esters mit einem Poly-C₂-C₄-alkylenglykol oder einem Poly-C₂-C₄-alkylenglykolmono-C₁-C₂₂-alkylether vor. Derartige Ester lassen sich durch die allgemeinen Formeln la und Ib beschreiben: 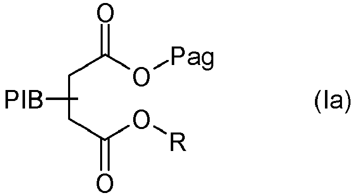 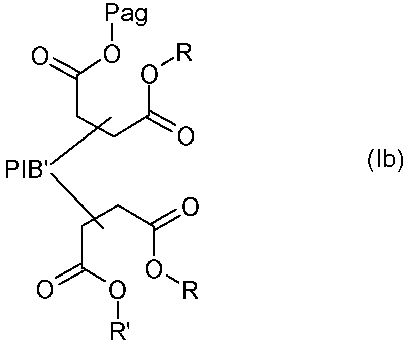 worin PIB und PIB' die zuvor für Formeln IIa und IIb angegebenen Bedeutungen aufweisen, R und R' unabhängig voneinander für Wasserstoff oder Pag stehen und Pag für einen von einem Poly-C₂-C₄-alkylenglykol oder einem Poly-C₂-C₄-alkylenglykolmono-C₁-C₂₂-alkylether abgeleiteten Rest stehen. In den Formeln la und Ib steht R insbesondere für Wasserstoff.

Unter Poly-C₂-C₄-alkylenglykolen versteht man lineare oder verzweigte Oligomere oder Polymere, die im Wesentlichen aus Wiederholungseinheiten der Formel -A-O- (im Folgenden auch Alkylenoxid-Wiederholungseinheiten) aufgebaut sind, worin A für C₂-C₄-Alkandiyl steht, und die an ihren Termini Hydroxylgruppen aufweisen.

Unter Poly-C₂-C₄-alkylenglykolmono-C₁-C₂₂-alkylethern versteht man lineare oder verzweigte Oligomere oder Polymere, die im Wesentlichen aus Wiederholungseinheiten der Formel -A-O-aufgebaut sind, worin A für C₂-C₄-Alkandiyl steht, die an einem ihrer Enden eine über Sauerstoff gebundene C₁-C₂₂-Alkylgruppe aufweisen und die an dem anderen Terminus bzw. den anderen Termini Hydroxylgruppen aufweisen.

In diesen Poly-C₂-C₄-alkylenglykolen bzw. Poly-C₂-C₄-alkylenglykolmono-C₁-C₂₂-alkylethern können die Wiederholungseinheiten der Formel -A-O- gleich oder verschieden sein. Sofern die Poly-C₂-C₄-alkylenglykole bzw. Poly-C₂-C₄-alkylenglykolmono-C₁-C₂₂-alkylether verschiedene Wiederholungseinheiten der Formel -A-O- aufweisen, können diese statistisch, alternierend oder in mehreren, z.B. 2, 3 oder 4 Blöcken angeordnet sein. In einer bestimmten Ausführungsform der Erfindung weisen die von den Poly-C₂-C₄-alkylenglykolen bzw. Poly-C₂-C₄-alkylenglykolmono-C₁-C₂₂-alkylethern unterschiedliche Wiederholungseinheiten der Formel -A-O- auf, die statistisch angeordnet sind.

C₂-C₄-Alkandiyl steht in diesem Zusammenhang für einen gesättigten divalenten Kohlenwasserstoffrest mit 2 bis 4 C-Atomen wie 1,2-Ethandiyl, 1,2-Propandiyl, 1,3-Propandiyl, 1,4-Butandiyl, 1,2-Butandiyl, 1,3-Butandiyl, 2,3-Butandiyl oder 1-Methyl-1,2-propandiyl.

C₁-C₂₂-Alkyl steht in diesem Zusammenhang für einen gesättigten, acyclischen monovalenten Kohlenwasserstoffrest mit 1 bis 22 C-Atomen, insbesondere mit 1 bis 8 C-Atomen oder 1 bis 4 C-Atomen wie Methyl, Ethyl, n-Propyl, Isopropyl, 1-Butyl, 2-Butyl, tert.-Butyl, Isobutyl, n-Pentyl, Isopentyl, n-Hexyl, Isohexyl, n-Heptyl, Isoheptyl, n-Octyl, Isooctyl, 2-Ethylhexyl, n-Nonyl, Isononyl, n-Decyl, 2-Propylheptyl, n-Undecyl, n-Dodecyl, n-Tridecyl, Myristyl, Pentadecyl, Palmityl (= Cetyl), Heptadecyl, Octadecyl, Nonadecyl, Arachinyl oder Behenyl.

Unter von Isobuten abgeleiteten Polymerresten, im Folgenden auch Polyisobutenyl-Reste, versteht man organische Reste, die von linearen oder verzweigten Oligomeren bzw. Polymeren des Isobutens abgeleitet sind und die bis zu 20 Gew.-%, vorzugsweise nicht mehr als 10 Gew.-% von Isobuten verschiedene C₂-C₁₂-Olefine, wie 1-Buten, 2-Buten, 2-Methyl-1-buten, 2-Methylpenten-1, 2-Methylhexen-1, 2-Ethylpenten-1, 2-Ethylhexen-1, 2-propylhepten-1, einpolymerisiert enthalten können. Derartige Reste können im Falle einwertiger Reste PIB beispielsweise durch die folgenden Formeln 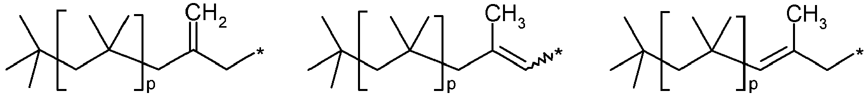 bzw. im Falle zweiwertiger Reste PIB' beispielsweise durch die folgenden Formeln beschrieben werden 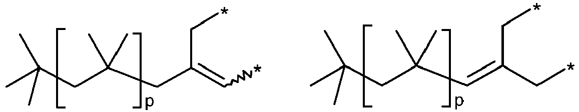 worin der Wert p+2 dem Polymerisationsgrad enspricht und die Anzahl an Isobuten-Einheiten im Polyisobuten-Rest angibt und * die Anknüpfung an den Bernsteinsäure(ester)-Rest bedeutet. In diesen Formeln kann ein Teil der Isobuteneinheiten -CH₂C(CH₃)₂-, in der Regel nicht mehr als 20 Gew.-%, vorzugsweise nicht mehr als 10 Gew.-%, durch davon verschiedene, von C₂-C₁₂-Olefinen abgeleitete C₂-C₁₂-Alkan-1,2-diylgruppen ersetzt sein. Der Polymerisationsgrad p+2 liegt typischerweise im Bereich von 5 bis 100, insbesondere im Bereich von 8 bis 80 und speziell im Bereich von 15 bis 65.

Im Hinblick auf die erfindungsgemäße Verwendung in Hydrogelen sind solche Ester der Polyisobutenbernsteinsäure bevorzugt, die, bezogen auf das Gesamtgewicht des Esters, zu wenigstens 50 Gew.-%, insbesondere zu wenigstens 70 Gew.-% aus Estern der Formel la bestehen. Vorzugsweise enthalten die Ester der Polyisobutenbernsteinsäure, bezogen auf das Gesamtgewicht des Esters, weniger als 30 Gew.-%, insbesondere weniger als 20 Gew.-% Ester der Formel Ib.
Im Hinblick auf die erfindungsgemäße Verwendung in Hydrogelen sind solche Ester der Polyisobutenbernsteinsäure bevorzugt, deren Polyisobutenrest des Esters ein zahlenmittleres Molekulargewicht im Bereich von 500 bis 5000 Dalton, insbesondere im Bereich von 800 bis 3600 aufweist.

In einer speziellen Ausführungsform der Erfindung weisen Polyisobutenreste der Polyisobutenbernsteinsäureester eine enge Molekulargewichtsverteilung auf. Die Polydispersität beträgt dann vorzugsweise höchstens 1,4, besonders bevorzugt höchstens 1,3, insbesondere höchstens 1,2. Unter Polydispersität versteht man den Quotienten aus gewichtsmittlerem Molekulargewicht M_{w} und zahlenmittlerem Molekulargewicht Mₙ (PDI = M_{w}/Mₙ).

Im Hinblick auf die erfindungsgemäße Verwendung in Hydrogelen sind solche Ester der Polyisobutenbernsteinsäure bevorzugt, die mit einem unter Poly-C₂-C₄-alkylenglykolen Poly-C₂-C₄-alkylenglykolmono-C₁-C₂₂-alkylether ausgewählten Alkohol oder einem Gemisch dieser Alkohole verestert sind, wobei der bzw. die Alkohole ein zahlenmittleres Molekulargewicht im Bereich von 500 bis 15000 Dalton, insbesondere im Bereich von 800 bis 10000 Dalton und speziell im Bereich von 1200 bis 5000 Dalton aufweist bzw. aufweisen.

Weiterhin hat es sich als vorteilhaft erwiesen, wenn der Alkohol, welcher mit der Polyisobutenbernsteinsäure verestert ist, unverzweigt ist, d.h. unter linearen Poly-C₂-C₄-alkylenglykolen und linearen Poly-C₂-C₄-alkylenglykolmono-C₁-C₂₀-alkylethern ausgewählt ist. Unverzweigte, d. h. lineare Poly-C₂-C₄-alkylenglykole und lineare Poly-C₂-C₄-alkylenglykolmono-C₁-C₂₀-alkylether können durch die folgende Formel (III) beschrieben werden:

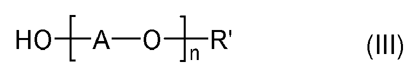

Hierin steht A für C₂-C₄-Alkandiyl wie zuvor definiert, das gleich oder verschieden sein kann und das vorzugsweise ausgewählt ist unter 1,2-Ethandiyl und 1,2-Propandiyl. R' steht für Wasserstoff oder C₁-C₂₂-Alkyl, insbesondere für Wasserstoff oder C₁-C₁₀-Alkyl und speziell für Wasserstoff oder C₁-C₄-Alkyl, z.B. für Methyl. Die Variable n gibt die mittlere Anzahl an Wiederholungseinheiten [A-O] an (Zahlenmittel) und liegt typischerweise im Bereich von 10 bis 350, insbesondere im Bereich von 15 bis 200.

Dementsprechend steht der Rest Pag in den Formeln la und Ib vorzugsweise für einen Rest der Formel 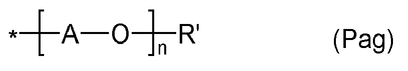 worin A, R und n die zuvor angegebenen Bedeutungen aufweisen und * die Verküpfung zum Sauerstoffatom des Polyisbutenbernsteinsäurerestes bedeutet.

In Formel III bzw. in Formel Pag können die Wiederholungseinheiten der Formel -A-O- gleich oder verschieden sein. Sofern die Formeln III bzw. in Formeln Pag verschiedene Wiederholungseinheiten der Formel -A-O- aufweisen, können diese statistisch oder in mehreren, z.B. 2, 3 oder 4 Blöcken angeordnet sein. In einer bestimmten Ausführungsform der Erfindung weisen die Formeln III bzw. in Formeln Pag unterschiedliche Wiederholungseinheiten der Formel - A-O- auf, die statistisch angeordnet sind.

Weiterhin hat es sich als vorteilhaft erwiesen, wenn der Alkohol, welcher mit der Polyisobutenbernsteinsäure verestert ist, zu wenigstens 50 mol-%, und insbesondere zu wenigstens 70 mol-%, bezogen auf die Gesamtzahl an Alkylenoxid-Wiederholungseinheiten im Alkohol, aus Wiederholungseinheiten der Formel [CH₂CH₂O] aufgebaut ist. Dementsprechend beträgt in den Formeln III und Pag der Anteil an Wiederholungseinheiten der Formel [CH₂CH₂O] wenigstens 50 mol-%, und insbesondere wenigstens 70 mol-%, bezogen auf die Gesamtzahl an Wiederholungseinheiten A-O.

In einer speziellen Ausführungsform der Erfindung sind alle oder nahezu alle Wiederholungseinheiten A-O des Poly-C₂-C₄-alkylenglykole bzw. des Poly-C₂-C₄-alkylenglykolmono-C₁-C₂₀-alkylethers, bzw. alle oder nahezu alle Wiederholungseinheiten A-O in den Formeln III und Pag, Wiederholungseinheiten der Formel [CH₂CH₂O].

In einerweiteren bevorzugten Ausführungsform der Erfindung umfasst der Alkohol, welcher mit der Polyisobutenbernsteinsäure verestert ist, insbesondere der Alkohol der Formel III bzw. der Rest Pag
- 50 mol-% bis 99 mol-%, und insbesondere 70 mol-% bis 98 mol-%, bezogen auf die Gesamtzahl an Alkylenoxid-Wiederholungseinheiten im Alkohol, Wiederholungseinheiten der Formel [CH₂CH₂O], sowie
- 1 mol-% bis 50 mol-%, und insbesondere 2 mol-% bis 30 mol-%, bezogen auf die Gesamtzahl an Alkylenoxid-Wiederholungseinheiten im Alkohol, Wiederholungseinheiten der Formel [A'-O], worin A' für C₃-C₄-Alkandiyl steht, und insbesondere Wiederholungseinheiten der Formel [CH₂CH(CH₃)O].
In einer speziellen Ausgestaltung dieser bevorzugten Ausführungsform sind die voneinander verschiedenen Wiederholungseinheiten [CH₂CH₂O] und [A'-O] nicht blockartig sondern statistisch verteilt oder alternierend angeordnet.
Ferner hat es sich als vorteilhaft erwiesen, wenn der Alkoholbestandteil und die dem Ester zugrunde liegende Polyisobutenbernsteinsäure so ausgewählt ist, dass der Ester im Mittel ein Gewichtsverhältnis von Polyisobutenrest zu Alkoholrest im Bereich von 10:1 bis 1:30, vorzugsweise im Bereich von 1,5:1 bis 1:20 und insbesondere im Bereich von 1:1 bis 1:10 aufweist. Weiterhin enthält die erfindungsgemäße Zusammensetzung mindestens ein Tensid aus der Gruppe bestehend aus Alkylpolyglycosiden, Amphoacetaten, Amphodiacetaten, Betainen, Fettalkoholethercarbonsäuren, Fettsäuresarcosinaten, Cocoamidopropylaminoxid, Aminopropionaten und Biotensiden.

Die Menge der Tenside ist in den Ansprüchen 3-4 offenbart.

Alkylpolyglykoside sind nichtionische Tenside, die durch die Reaktion von Zuckern und Alkoholen nach den einschlägigen Verfahren der präparativen organischen Chemie erhalten werden können, wobei es je nach Art der Herstellung zu einem Gemisch monoalkylierter, oligomerer oder polymerer Zucker kommt. Bevorzugte Alkylpolyglykoside sind die Alkylpolyglucoside, wobei besonders bevorzugt der Alkohol ein langkettiger Fettalkohol oder ein Gemisch langkettiger Fettalkohole mit verzweigten oder unverzweigten C₈- bis C₁₈-Alkylketten ist und der Oligomerisierungsgrad (DP) der Zucker zwischen 1 und 10, vorzugsweise 1 bis 6, insbesondere 1,1 bis 3, äußerst bevorzugt 1,1 bis 1,7, beträgt, beispielsweise C₈₋₁₀-Alkyl-1.5-glucosid (DP von 1,5).
Amphoacetate, Amphodiacetate und Betaine zählen zu den Amphotensiden. Bevorzugt werden hierbei Cocoamphodiacetat und/oder Cocoamidopropylbetain eingesetzt.
Bei den Fettsäuresarcosinaten handelt es sich um die Kondensationsprodukte von Fettsäuren mit Sarcosin (N-Methylglycin). Sie zählen ebenso wie die Fettalkoholethercarbonsäuren zu den Aniontensiden.
Zu den Biotensiden zählen beispielsweise die Saponine oder auch die Glycolipide. Glycolipide im engeren Sinn sind Verbindungen, in denen eine oder mehrere Monosaccharideinheiten glycosidisch mit einem Lipidanteil verbunden sind. Bevorzugte Glycolipide sind Sophorolipide, Rhamnolipide, Glucoselipide, Cellobioselipide, Trehaloselipide sowie Gemische derselben.

### Parfüm

Die erfindungsgemäße Zusammensetzung enthält einen oder mehrere Duftstoffe, vorzugsweise in einer Menge von 0,01 bis 15 Gew.-%, insbesondere 0,05 bis 10 Gew.-%, besonders bevorzugt 0,1 bis 8 Gew.-%. Als eine Parfümkomponente kann dabei d-Limonen enthalten sein. In einer besonders bevorzugten Ausführungsform enthält die Zusammensetzung dabei ein Parfüm aus ätherischen Ölen (auch als essentielle Öle bezeichnet). Als solche sind beispielsweise Pine-, Citrus-, Jasmin-, Patchouly-, Rosen- oder Ylang-Ylang-Öl im Sinne dieser Erfindung einsetzbar. Ebenfalls geeignet sind Muskateller-Salbeiöl, Kamillenöl, Lavendelöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Haftfeste Riechstoffe, die im Rahmen der vorliegenden Erfindung vorteilhafterweise in den Parfümölen einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lemongrasöl, Moschuskörneröl, Myrrhenöl, Nelkenöl, Neroliöl, Niaouliöl, Olibanumöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Sternanisöl, Thujaöl, Thymianöl, Verbenaöl, Vetiveröl, Wacholderbeeröl, Wermutöl, Wintergrünöl, Ylang -Ylang-Öl, Ysop-Öl, Zimtöl, Zimtblätteröl sowie Zypressenöl.

Aber auch die höhersiedenden bzw. festen Riechstoffe natürlichen oder synthetischen Ursprungs können im Rahmen der vorliegenden Erfindung vorteilhafterweise als haftfeste Riechstoffe bzw. Riechstoffgemische in den Parfümölen eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Borneol, Bornylacetat, α-Bromstyrol, n-Decylaldehyd, n-Dodecyl-aldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Di-methylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p- Kresolmethyl-ether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimtalkohol, Zimtsäure, Zimtsäureethylester, Zimtsäurebenzylester.

Zu den leichter flüchtigen Riechstoffen, die im Rahmen der vorliegenden Erfindung in den Parfümölen vorteilhaft einsetzbar sind, zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Citral, Citronellal.

### Weitere Inhaltsstoffe

Neben den bisher genannten Komponenten kann die erfindungsgemäße Zusammensetzung weitere übliche Inhaltsstoffe von WC-Reinigungsmitteln enthalten, vorzugsweise ausgewählt aus der Gruppe umfassend Säuren, Basen, Salze, Verdickungsmittel, antimikrobielle Wirkstoffe, Konservierungsstoffe, Komplexbildner, Polymere, Farbstoffe, Parfümbooster, Füllstoffe, Builder, Bleichmittel, Bitterstoffe, Korrosionsinhibitoren, Enzyme, Mikroorganismen, Wirkstoffe zur Biofilmentfernung, Wirkstoffe zur Inhibierung der Kalkablagerung, Wirkstoffe zur Verminderung der Schmutzhaftung sowie Gemische derselben. Insgesamt sollten nicht mehr als 60 Gew.-% weitere Inhaltsstoffe enthalten sein, vorzugsweise 0,01 bis 40 Gew.-%, insbesondere 0,2 bis 35 Gew.-%.

### Säuren

Erfindungsgemäße Zusammensetzungen können zur Verstärkung der Reinigungsleistung gegenüber Kalk und Urinstein eine oder mehrere Säuren und/oder deren Salze enthalten. Bevorzugt werden die Säuren aus nachwachsenden Rohstoffen hergestellt. Als Säuren eignen sich daher insbesondere organische Säuren wie Essigsäure, Citronensäure, Glycolsäure, Milchsäure, Bernsteinsäure, Adipinsäure, Äpfelsäure, Weinsäure und Gluconsäure sowie Gemische derselben. Daneben kann aber auch Amidosulfonsäure eingesetzt werden. Besonders bevorzugt sind die Säuren und/oder ihre Salze ausgewählt aus der Gruppe umfassend Citronensäure, Milchsäure, Amidosulfonsäure, ihre Salze sowie Gemische derselben. Sie werden vorzugsweise in Mengen von 0,01 bis 10 Gew.-% eingesetzt, besonders bevorzugt 0,2 bis 5 Gew.-%.

Daneben kann die Zusammensetzung in einer Ausführungsform anorganische Salze enthalten, vorzugsweise Alkali- oder Erdalkalimetallsalze, insbesondere Carbonate, Sulfate, Halogenide oder Phosphate sowie Gemische derselben. Besonders bevorzugt werden Natriumsulfat und/oder Natriumcarbonat eingesetzt. Natriumsulfat kann dabei in einer Menge von bis zu 60 Gew.-% enthalten sein, vorzugsweise 0,01 bis 60 Gew.-%, besonders bevorzugt 20 bis 60 Gew.-%, insbesondere 35 bis 55 Gew.-%. Natriumcarbonat und weitere Salze können in einer Menge von bis zu 30 Gew.-%, vorzugsweise bis zu 10 Gew.-%, besonders bevorzugt bis zu 5 Gew.-% enthalten sein.

### Basen

In erfindungsgemäßen Zusammensetzungen können weiterhin Alkalien enthalten sein. Als Basen werden in erfindungsgemäßen Mitteln vorzugsweise solche aus der Gruppe der Alkali- und Erdalkalimetallhydroxide und -carbonate, insbesondere Natriumcarbonat oder Natriumhydroxid, eingesetzt. Daneben können aber auch Ammoniak und/oder Alkanolamine mit bis zu 9 C-Atomen im Molekül verwendet werden, vorzugsweise die Ethanolamine, insbesondere Monoethanolamin.

### Antimikrobielle Wirkstoffe

Eine besondere Form der Reinigung stellen die Desinfektion und die Sanitation dar. In einer entsprechenden besonderen Ausführungsform der Erfindung enthält das Gel oder die Paste daher einen oder mehrere antimikrobielle Wirkstoffe, vorzugsweise in einer Menge von bis zu 40 Gew.-%, bevorzugt 0,01 bis 25 Gew.-%, insbesondere 0,1 bis 5 Gew.-%.

Die Begriffe Desinfektion, Sanitation, antimikrobielle Wirkung und antimikrobieller Wirkstoff haben im Rahmen der erfindungsgemäßen Lehre die fachübliche Bedeutung. Während Desinfektion im engeren Sinne der medizinischen Praxis die Abtötung von - theoretisch allen - Infektionskeimen bedeutet, ist unter Sanitation die möglichst weitgehende Eliminierung aller- auch der für den Menschen normalerweise unschädlichen saprophytischen - Keime zu verstehen. Hierbei ist das Ausmaß der Desinfektion bzw. Sanitation von der antimikrobiellen Wirkung des angewendeten Mittels abhängig, die mit abnehmendem Gehalt an antimikrobiellem Wirkstoff bzw. zunehmender Verdünnung des Mittels zur Anwendung abnimmt.

Erfindungsgemäß geeignet sind beispielsweise antimikrobielle Wirkstoffe aus den Gruppen der Alkohole, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff-, Stickstoff-Acetale sowie -Formale, Benzamidine, Isothiazole und deren Derivate wie Isothiazoline und Isothiazolinone, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propynyl-butyl-carbamat, lod, lodophore, Aktivchlor abspaltenden Verbindungen und Peroxide. Bevorzugte antimikrobielle Wirkstoffe werden vorzugsweise ausgewählt aus der Gruppe umfassend 1,3-Butandiol, Phenoxyethanol, 1,2-Propylenglykol, Glycerin, Undecylensäure, Citronensäure, Milchsäure, Benzoesäure, Salicylsäure, Thymol, 2-Benzyl-4-chlorphenol, 2,2'-Methylen-bis-(6-brom-4-chlorphenol), 2,4,4'-Trichlor-2'-hydroxydiphenylether, N-(4-Chlorphenyl)-N-(3,4-dichlorphenyl)-harnstoff, N,N'-(1,10-decandiyldi-1-pyridinyl-4-yliden)-bis-(1-octanamin)-dihydrochlorid, N,N'-Bis-(4-Chlorphenyl)-3,12-diimino-2,4,11,13-tetraazatetradecandiimidamid, antimikrobielle quaternäre oberflächenaktive Verbindungen, Guanidine, Trichloroisocyanursäure und Natrium-Dichlorisocyanurat (DCI, 1,3-Dichlor-5H-1,3,5-triazin-2,4,6-trion Natriumsalz). Bevorzugte antimikrobiell wirkende oberflächenaktive quaternäre Verbindungen enthalten eine Ammonium-, Sulfonium-, Phosphonium-, Jodonium- oder Arsoniumgruppe. Weiterhin können auch antimikrobiell wirksame ätherische Öle eingesetzt werden, die gleichzeitig für eine Beduftung des Reinigungsmittels sorgen. Besonders bevorzugte antimikrobielle Wirkstoffe sind jedoch ausgewählt aus der Gruppe umfassend Salicylsäure, quaternäre Tenside, insbesondere Benzalkoniumchlorid, PeroxoVerbindungen, insbesondere Natriumpercarbonat oder Phthalimidoperoxyhexanoic acid, Alkalimetallhypochlorit, Trichloroisocyanursäure, Natriumdichlorisocyanurat sowie Gemische derselben.

### Konservierungsstoffe

Konservierungsstoffe können gleichfalls in erfindungsgemäßen Zusammensetzungen enthalten sein. Als solche können im Wesentlichen die bei den antimikrobiellen Wirkstoffen genannten Stoffe eingesetzt werden.

### Komplexbildner

Komplexbildner (*INCI* Chelating Agents), auch Sequestriermittel genannt, sind Inhaltsstoffe, die Metallionen zu komplexieren und inaktivieren vermögen, um ihre nachteiligen Wirkungen auf die Stabilität oder das Aussehen der Mittel, beispielsweise Trübungen, zu verhindern. Einerseits ist es dabei wichtig, die mit zahlreichen Inhaltsstoffen inkompatiblen Calcium- und Magnesiumionen der Wasserhärte zu komplexieren. Die Komplexierung der Ionen von Schwermetallen wie Eisen oder Kupfer verzögert andererseits die oxidative Zersetzung der fertigen Mittel. Zudem unterstützen die Komplexbildner die Reinigungswirkung.

Geeignet sind beispielsweise die folgenden gemäß *INCI* bezeichneten Komplexbildner: Aminotrimethylene Phosphonic Acid, Beta-Alanine Diacetic Acid, Calcium Disodium EDTA, Citric Acid, Cyclodextrin, Cyclohexanediamine Tetraacetic Acid, Diammonium Citrate, Diammonium EDTA, Diethylenetriamine Pentamethylene Phosphonic Acid, Dipotassium EDTA, Disodium Azacycloheptane Diphosphonate, Disodium EDTA, Disodium Pyrophosphate, EDTA, Etidronic Acid, Galactaric Acid, Gluconic Acid, Glucuronic Acid, HEDTA, Hydroxypropyl Cyclodextrin, Methyl Cyclodextrin, Pentapotassium Triphosphate, Pentasodium Aminotrimethylene Phosphonate, Pentasodium Ethylenediamine Tetramethylene Phosphonate, Pentasodium Pentetate, Pentasodium Triphosphate, Pentetic Acid, Phytic Acid, Potassium Citrate, Potassium EDTMP, Potassium Gluconate, Potassium Polyphosphate, Potassium Trisphosphonomethylamine Oxide, Ribonic Acid, Sodium Chitosan Methylene Phosphonate, Sodium Citrate, Sodium Diethylenetriamine Pentamethylene Phosphonate, Sodium Dihydroxyethylglycinate, Sodium EDTMP, Sodium Gluceptate, Sodium Gluconate, Sodium Glycereth-1 Polyphosphate, Sodium Hexametaphosphate, Sodium Metaphosphate, Sodium Metasilicate, Sodium Phytate, Sodium Polydimethylglycinophenolsulfonate, Sodium Trimetaphosphate, TEA-EDTA, TEA-Polyphosphate, Tetrahydroxyethyl Ethylenediamine, Tetrahydroxypropyl Ethylenediamine, Tetrapotassium Etidronate, Tetrapotassium Pyrophosphate, Tetrasodium EDTA, Tetrasodium Etidronate, Tetrasodium Pyrophosphate, Tripotassium EDTA, Trisodium Dicarboxymethyl Alaninate, Trisodium EDTA, Trisodium HEDTA, Trisodium NTA und Trisodium Phosphate.

### Polymere

Das erfindungsgemäße WC-Reinigungsgel kann weiterhin Polymere enthalten. Diese können beispielsweise zur Verringerung der Kalkbildung sowie der Wiederanschmutzungsneigung dienen. Bevorzugte Polymere sind dabei Acrylcopolymere, wie sie etwa von der Firma Rhodia unter dem Handelsnamen Mirapol kommerziell erhältlich sind.

### Farbstoffe

Als weitere Inhaltsstoffe kann die erfindungsgemäße Zusammensetzung einen oder mehrere Farbstoffe (*INCI* Colorants) enthalten. Als Farbstoffe können dabei sowohl wasserlösliche als auch öllösliche Farbstoffe verwendet werden, wobei einerseits die Kompatibilität mit weiteren Inhaltsstoffen, beispielsweise Bleichmitteln, zu beachten ist und andererseits der eingesetzte Farbstoff gegenüber der WC-Keramik auch bei längerem Einwirken nicht substantiv wirken sollte. Die Farbstoffe sind vorzugsweise in einer Menge von 0,0001 bis 5 Gew.-%, insbesondere 0,0005 bis 0,25 Gew.-%, besonders bevorzugt 0,0008 bis 0,08 Gew.-%, enthalten.

### Builder

In den erfindungsgemäßen Zusammensetzungen können ggf. wasserlösliche und/oder wasserunlösliche Builder eingesetzt werden. Dabei sind wasserlösliche Builder bevorzugt, da sie in der Regel weniger dazu tendieren, auf harten Oberflächen unlösliche Rückstände zu hinterlassen. Übliche Builder, die im Rahmen der Erfindung zugegen sein können, sind die niedermolekularen Polycarbonsäuren und ihre Salze, die homopolymeren und copolymeren Polycarbonsäuren und ihre Salze, die Citronensäure und ihre Salze, die Carbonate, Phosphate und Silikate. Zu wasserunlöslichen Buildern zählen die Zeolithe, die ebenfalls verwendet werden können, ebenso wie Mischungen der vorgenannten Buildersubstanzen.

### Bleichmittel

Erfindungsgemäß können Bleichmittel dem Reinigungsmittel zugesetzt werden. Geeignete Bleichmittel umfassen Peroxide, Persäuren und/oder Perborate, besonders bevorzugt ist Natriumpercarbonat oder Phthalimidoperoxyhexanoic acid. Chlorhaltige Bleichmittel wie Trichlorisocyanursäure oder Natriumdichlorisocyanurat sind dagegen bei sauer formulierten Reinigungsmitteln aufgrund der Freisetzung giftiger Chlorgas-Dämpfe weniger geeignet, können jedoch in alkalisch eingestellten Reinigungsmitteln eingesetzt werden. Unter Umständen kann neben dem Bleichmittel auch ein Bleichaktivator vonnöten sein.

### Korrosionsinhibitoren

Geeignete Korrosionsinhibitoren (*INCI* Corrosion Inhibitors) sind beispielsweise folgende gemäß *INCI* benannte Substanzen: Cyclohexylamine, Diammonium Phosphate, Dilithium Oxalate, Dimethylamino Methylpropanol, Dipotassium Oxalate, Dipotassium Phosphate, Disodium Phosphate, Disodium Pyrophosphate, Disodium Tetrapropenyl Succinate, Hexoxyethyl Diethylammonium, Phosphate, Nitromethane, Potassium Silicate, Sodium Aluminate, Sodium Hexametaphosphate, Sodium Metasilicate, Sodium Molybdate, Sodium Nitrite, Sodium Oxalate, Sodium Silicate, Stearamidopropyl Dimethicone, Tetrapotassium Pyrophosphate, Tetrasodium Pyrophosphate, Triisopropanolamine.

### Enzyme

Die Zusammensetzung kann auch Enzyme enthalten, vorzugsweise Proteasen, Lipasen, Amylasen, Hydrolasen und/oder Cellulasen. Sie können dem erfindungsgemäßen Mittel in jeder nach dem Stand der Technik etablierten Form zugesetzt werden. Hierzu gehören Lösungen der Enzyme, vorteilhafterweise möglichst konzentriert, wasserarm und/oder mit Stabilisatoren versetzt. Alternativ können die Enzyme verkapselt werden, beispielsweise durch Sprühtrocknung oder Extrusion der Enzymlösung zusammen mit einem, vorzugsweise natürlichen, Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil. Weiterhin können in enzymhaltigen Mitteln Enzymstabilisatoren vorhanden sein, um ein in einem erfindungsgemäßen Mittel enthaltenes Enzym vor Schädigungen wie beispielsweise Inaktivierung, Denaturierung oder Zerfall etwa durch physikalische Einflüsse, Oxidation oder proteolytische Spaltung zu schützen. Als Enzymstabilisatoren sind, jeweils in Abhängigkeit vom verwendeten Enzym, insbesondere geeignet: Benzamidin-Hydrochlorid, Borax, Borsäuren, Boronsäuren oder deren Salze oder Ester, vor allem Derivate mit aromatischen Gruppen, etwa substituierte Phenylboronsäuren beziehungsweise deren Salze oder Ester; Peptidaldehyde (Oligopeptide mit reduziertem C-Terminus), Aminoalkohole wie Mono-, Di-, Triethanol- und -Propanolamin und deren Mischungen, aliphatische Carbonsäuren bis zu C₁₂, wie Bernsteinsäure, andere Dicarbonsäuren oder Salze der genannten Säuren; endgruppenverschlossene Fettsäureamidalkoxylate; niedere aliphatische Alkohole und vor allem Polyole, beispielsweise Glycerin, Ethylenglykol, Propylenglykol oder Sorbit; sowie Reduktionsmittel und Antioxidantien wie Natriumsulfit und reduzierende Zucker. Weitere geeignete Stabilisatoren sind aus dem Stand der Technik bekannt. Bevorzugt werden Kombinationen von Stabilisatoren verwendet, beispielsweise die Kombination aus Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen.

Die Zusammensetzung wird vorteilhafterweise mit einem Applikator aufgetragen. Es kann aus ästhetischen Gründen, aber auch, um miteinander unverträgliche Wirkstoffe gemeinsam zur Anwendung bringen zu können, wünschenswert sein, gleichzeitig zwei oder sogar mehr Portionen des erfindungsgemäßen haftenden Gels oder der Paste nebeneinander auf die WC-Keramik aufzutragen. Hierzu eignet sich in besonderer Weise ein Zweikammer-Applikator, wie er auch in der Patentanmeldung DE 102010028352.5 beschrieben wird. Dieser Applikator kann in seinen beiden Kammern Gele oder Pasten gleicher oder unterschiedlicher Zusammensetzung bevorraten.

Bei der Anwendung wird mit dem Applikator üblicherweise eine Menge von 4 bis 8 g der Zusammensetzung auf die WC-Keramik aufgetragen. Diese Auftragsmenge ist vorteilhafterweise nach 100 bis 200 Spülvorgängen vollständig abgelöst.

Das erfindungsgemäße Mittel wird hergestellt, indem die einzelnen Komponenten gemischt und anschließend abgefüllt werden. Als Mischer kommen dabei alle Maschinen in Frage, die viskose Massen verarbeiten können, beispielsweise SpeedMixer, Planetenmischer oder Statikmischer. Um Verluste an flüchtigen Inhaltsstoffen, insbesondere beim Parfüm, so gering wie möglich zu halten, erfolgt die Verarbeitung bei möglichst niedriger Temperatur, maximal aber bei 85°C. Bei dieser Temperatur wird das Polymer aufgeschmolzen und unter Rühren im Mischer nacheinander Tenside, Parfümöl und weitere Komponenten zugegeben. Im warmen Zustand ist die Formulierung fließfähig und kann abgefüllt werden.

### Ausführungsbeispiele

Es wurden zwei gelförmige Formulierungen E1 und E2 hergestellt, die als mehrphasiges Mittel in einen Zweikammerapplikator abgefüllt wurden. Hierbei diente E1 vor allem zur Beduftung, während E2 eine Reinigungsformel darstellt. Die Zusammensetzungen sind der nachfolgenden Tabelle zu entnehmen. Die Mengenangaben sind dabei in Gew.-% Aktivsubstanz.

| | E1 | E2 |
|---|---|---|
| Ester der Polyisobutenbernsteinsäure | 46,00 | 46,00 |
| Alkylpolyglucosid | 9,10 | 9,10 |
| Cocoamidopropylbetain | 6,29 | 6,29 |
| Parfümöl | 4,90 | 4,90 |
| Bitterstoff (Bitrex) | 0,0010 | 0,0010 |
| Trinatriumcitrat | -- | 0,10 |
| Farbstoff gelb | 0,0150 | -- |
| Farbstoff blau | -- | 0,0130 |
| Wasser (vollentsalzt) | ad 100 | ad 100 |

## Patentansprüche

1. Haftende gelförmige oder pastöse Zusammensetzung zur Reinigung und/oder Beduftung eines WCs, welche innen auf die WC-Keramik aufgetragen und erst nach mehreren Spülvorgängen abgespült wird, enthaltend
- mindestens ein Tensid aus der Gruppe bestehend aus Alkylpolyglycosiden, Amphoacetaten, Amphodiacetaten, Betainen, Fettalkoholethercarbonsäuren, Fettsäuresarcosinaten, Cocoamidopropylaminoxid, Aminopropionaten und Biotensiden,
- Parfüm und
- Wasser,
**dadurch gekennzeichnet, dass** sie als Haftvermittler einen Ester der Polyisobutenbernsteinsäure enthält und frei von weiteren Haftvermittlern und Tensiden ist.

2. Haftende Zusammensetzung gemäß Anspruch 1, **dadurch gekennzeichnet, dass** der Ester der Polyisobutenbernsteinsäure in einer Menge von 20 bis 80 Gew.-%, vorzugsweise 30 bis 70 Gew.-%, besonders bevorzugt 40 bis 60 Gew.-% enthalten ist.

3. Haftende Zusammensetzung gemäß einem der Ansprüche 1 und 2, **dadurch gekennzeichnet, dass** sie Alkylpolyglycosid in einer Menge von 0 bis 40 Gew.-%, vorzugsweise 2 bis 35 Gew.-%, besonders bevorzugt 5 bis 20 Gew.-% enthält.

4. Haftende Zusammensetzung gemäß einem der Ansprüche 1 und 2, **dadurch gekennzeichnet, dass** sie mindestens ein Tensid aus der Gruppe bestehend aus Amphoacetaten, Amphodiacetaten, Betainen, Fettalkoholethercarbonsäuren, Fettsäuresarcosinaten, Cocoamidopropylaminoxid, Aminopropionaten und Biotensiden in einer Menge von 0 bis 40 Gew.-%, vorzugsweise 2 bis 35 Gew.-%, besonders bevorzugt 3 bis 20 Gew.-% enthält.

5. Haftende Zusammensetzung gemäß einem der Ansprüche 1 und 2, **dadurch gekennzeichnet, dass** sie eine Kombination aus Alkylpolyglycosid und mindestens einem Tensid aus der Gruppe bestehend aus Amphoacetaten, Amphodiacetaten, Betainen, Fettalkoholethercarbonsäuren, Fettsäuresarcosinaten, Cocoamidopropylaminoxid, Aminopropionaten und Biotensiden enthält.

6. Haftende Zusammensetzung gemäß einem der vorangehenden Ansprüche, **dadurch gekennzeichnet, dass** sie weitere übliche Inhaltsstoffe von WC-Reinigungsmitteln enthält, vorzugsweise ausgewählt aus der Gruppe umfassend Säuren, Basen, Salze, Verdickungsmittel, antimikrobielle Wirkstoffe, Konservierungsstoffe, Komplexbildner, Farbstoffe, Parfümbooster, Füllstoffe, Builder, Bleichmittel, Bitterstoffe, Korrosionsinhibitoren, Enzyme, Mikroorganismen, Wirkstoffe zur Biofilmentfernung, Wirkstoffe zur Inhibierung der Kalkablagerung, Wirkstoffe zur Verminderung der Schmutzhaftung sowie Gemische derselben.

7. Haftende Zusammensetzung gemäß einem der vorangehenden Ansprüche, **dadurch gekennzeichnet, dass** eine Auftragsmenge von 4 bis 8 g nach 100 bis 200 Spülvorgängen vollständig abgelöst ist

8. Ein Verfahren zum Auftragen einer haftenden Zusammensetzung gemäß einem der Ansprüche 1 bis 7 auf eine WC-Keramik, **dadurch gekennzeichnet, dass** sie mittels eines Applikators auf die WC-Keramik aufgetragen wird und dort haftet.

9. Das Verfahren gemäß Anspruch 8, **dadurch gekennzeichnet, dass** mit dem Applikator gleichzeitig zwei Portionen nebeneinander aufgetragen werden.

10. Das Verfahren gemäß Anspruch 9, **dadurch gekennzeichnet, dass** der Applikator zwei Kammern aufweist, in denen Gele oder Pasten gleicher oder unterschiedlicher Zusammensetzung bevorratet werden.

## Claims

1. An adhesive gel or pasty composition for cleaning and/or fragrancing a toilet, which is applied to the inside of the toilet bowl and is only rinsed off after several flushing procedures, containing
- at least one surfactant from the group consisting of alkyl polyglycosides, amphoacetates, amphodiacetates, betaines, fatty alcohol ether carboxylic acids, fatty acid sarcosinates, cocoamidopropylamine oxide, aminopropionates and biosurfactants,
- perfume and
- water,
**characterized in that** it contains an ester of polyisobutene succinic acid as an adhesion promoter and is free of further adhesion promoters and surfactants.

2. The adhesive composition according to claim 1, **characterized in that** the ester of polyisobutene succinic acid is contained in an amount of from 20 to 80 wt.%, preferably from 30 to 70 wt.%, particularly preferably from 40 to 60 wt.%.

3. The adhesive composition to either claim 1 or claim 2, **characterized in that** it contains alkyl polyglycoside in an amount of from 0 to 40 wt.%, preferably from 2 to 35 wt.%, particularly preferably from 5 to 20 wt.%.

4. The adhesive composition according to either claim 1 or claim 2, **characterized in that** it contains at least one surfactant from the group consisting of amphoacetates, amphodiacetates, betaines, fatty alcohol ether carboxylic acids, fatty acid sarconisates, cocoamidopropylamine oxide, aminopropionates and biosurfactants in an amount of from 0 to 40 wt.%, preferably from 2 to 35 wt.%, particularly preferably from 3 to 20 wt.%.

5. The adhesive composition according to either claim 1 or claim 2, **characterized in that** it contains a combination of alkyl polyglycoside and at least one surfactant from the group consisting of amphoacetates, amphodiacetates, betaines, fatty alcohol ether carboxylic acids, fatty acid sarcosinates, cocoamidopropylamine oxide, aminopropionates and biosurfactants.

6. The adhesive composition according to one of the preceding claims, **characterized in that** it contains further conventional ingredients of toilet cleaning agents, preferably selected from the group comprising acids, bases, salts, thickening agents, antimicrobial active ingredients, preservatives, complexing agents, dyes, perfume boosters, fillers, builders, bleaching agents, bitter principles, corrosion inhibitors, enzymes, microorganisms, active ingredients for biofilm removal, active ingredients for inhibiting limescale deposition, active ingredients for reducing dirt adhesion, and mixtures thereof.

7. The adhesive composition according to one of the preceding claims, **characterized in that** an application quantity of from 4 to 8 g is completely dissolved after 100 to 200 flushing procedures.

8. A method for applying an adhesive composition according to one of claims 1 to 7 to a toilet bowl, **characterized in that** said composition is applied to the toilet bowl using an applicator and adheres to said toilet bowl.

9. The method according to claim 8, **characterized in that** two portions are simultaneously applied next to one another using the applicator.

10. The method according to claim 9, **characterized in that** the applicator comprises two chambers in which gels or pastes of the same or different compositions are stored.

## Revendications

1. Composition adhésive sous forme de gel ou de pâte pour le nettoyage et/ou la diffusion de parfum dans les WC, en application intérieure sur la céramique des WC et qui sera évacuée uniquement après plusieurs chasses d'eau, contenant
- au moins un tensioactif appartenant au groupe constitué des alkylpolyglucosides, des amphoacétates, des amphodiacétates, des bétaïnes, des acides carboxyliques d'éther d'alcool gras, des sarcosinates d'acide gras, le cocoamidopropylaminoxyde, les aminopropionates et des agents tensioactifs biologiques,
- du parfum et
- de l'eau,
**caractérisée en ce qu'**elle contient, en tant qu'agent adhésif, un ester de l'acide de polyisobutène succinique, la composition ne contenant aucun autre agent adhésif et autre tensioactif.

2. Composition adhésive selon la revendication 1, **caractérisée en ce que** l'ester de l'acide de polyisobutène succinique est présent dans une quantité allant de 20 à 80 % en poids, préférablement 30 à 70 % en poids, très préférablement 40 à 60 % en poids.

3. Composition adhésive selon l'une quelconque des revendications 1 et 2, **caractérisée en ce qu'**elle contient des alkylpolyglucosides dans une quantité allant de 0 à 40 % en poids, préférablement 2 à 35 % en poids, très préférablement 5 à 20 % en poids.

4. Composition adhésive selon l'une quelconque des revendications 1 et 2, **caractérisée en ce qu'**elle contient au moins un tensioactif issu du groupe constitué des amphoacétates, des amphodiacétates, des bétaïnes, des acides carboxyliques d'éther d'alcool gras, des sarcosinates d'acide gras, du cocoamidopropylaminoxyde, des aminopropionates et des agents tensioactifs biologiques dans une quantité allant de 0 à 40 % en poids, préférablement 2 à 35 % en poids, très préférablement 3 à 20 % en poids.

5. Composition adhésive selon l'une quelconque des revendications 1 et 2, **caractérisée en ce qu'**elle contient une combinaison d'alkylpolyglucosides et d'au moins un tensioactif issu du groupe constitué des amphoacétates, des amphodiacétates, des bétaïnes, des acides carboxyliques d'éther d'alcool gras, des sarcosinates d'acide gras, du cocoamidopropylaminoxyde, des aminopropionates et des agents tensioactifs biologiques.

6. Composition adhésive selon l'une quelconque des revendications précédentes, **caractérisée en ce qu'**elle contient d'autres composants usuels des produits nettoyants pour WC, préférablement sélectionnés dans le groupe contenant les acides, les bases, les sels, les agents épaississants, les principes actifs antimicrobiens, des agents conservateurs, des agents de chélation, des colorants, des amplificateurs de parfum, des agents de charge, des adjuvants, des agents blanchissants, des amérisants, des inhibiteurs de corrosion, des enzymes, des microorganismes, des principes actifs pour l'élimination du biofilm, des principes actifs pour l'inhibition des dépôts calcaires, des principes actifs pour la diminution de l'adhérence de la saleté, ainsi que des composés de ceux-ci.

7. Composition adhésive selon l'une quelconque des revendications précédentes, **caractérisée en ce qu'**une quantité étalée de 4 à 8 g sera complétement évacuée après 100 à 200 chasses d'eau.

8. Procédé d'étalage d'une composition adhésive selon l'une quelconque des revendications 1 à 7 sur de la céramique de WC, **caractérisée en ce qu'**elle est étalée sur la céramique des WC à l'aide d'un applicateur et qu'elle adhère à cet endroit.

9. Procédé selon la revendication 8, **caractérisé en ce que** l'applicateur permet d'étaler simultanément deux portions l'une à côté de l'autre.

10. Procédé selon la revendication 9, **caractérisé en ce que** l'applicateur possède deux chambres, dans lesquelles le gel ou la pâte de composition identique ou différente sont stockés.
